(19) Europäisches Patentamt
European Patent Office
Office européen des brevets

(11) Veröffentlichungsnummer: **0 050 225**
**A1**

(12) **EUROPÄISCHE PATENTANMELDUNG**

(21) Anmeldenummer: **81107483.0**

(22) Anmeldetag: **21.09.81**

(51) Int. Cl.³: **C 07 C 147/06,** C 07 C 147/107, C 07 C 149/30, C 07 D 521/00 // (C07D521/00, 235/06, 235/16, 235/18, 235/20, 263/56, 263/62, 271/10, 277/64, 285/12, 295/18, 307/38, 307/79, 413/04, 413/06, 413/10)

(30) Priorität: **17.10.80 DE 3039208**

(43) Veröffentlichungstag der Anmeldung: **28.04.82**
**Patentblatt 82/17**

(84) Benannte Vertragsstaaten: **CH DE FR GB IT LI**

(71) Anmelder: **BASF Aktiengesellschaft,**
**Carl-Bosch-Strasse 38, D-6700 Ludwigshafen (DE)**

(72) Erfinder: **Vamvakaris, Christos, Dr., Riedweg 6,**
**D-6701 Kallstadt (DE)**

(54) **Neue Sulfone, deren Herstellung und Verwendung.**

(57) Die Erfindung betrifft Sulfone der allgemeinen Formel

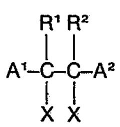

I,

in der

$A^2$ einen Rest $A^1$ oder einen Rest der Formel

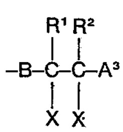

bedeutet, wobei

$A^1$ und $A^3$ unabhängig voneinander gegebenenfalls ein- oder mehrfach substituiertes Aryl oder Heteroaryl, Cyan, Carbonester, gegebenenfalls substituiertes Carbamoyl, Carboxyl, Alkanoyl oder Aroyl,

B eine direkte Bindung oder ein Brückenglied und

$R^1$ und $R^2$ unabhängig voneinander Wasserstoff, $C_1$- bis $C_4$-Alkyl, Aryl, Heteroaryl, Carboxyl, Cyan, Carbonester oder gegebenenfalls substituiertes Carbamoyl bedeuten, jeweils ein X Wasserstoff und das andere X ein Sulfonrest ist und wobei für $A^2$ gleich $A^1$ die beiden Reste $A^1$ gleich oder verschieden sein können.

Die erfindungsgemäßen Verbindungen sind wervolle Zwischenprodukte für Pharmaceutika und Farbstoffe.

EP 0 050 225 A1

ACTORUM AG

# Neue Sulfone, deren Herstellung und Verwendung

Die Erfindung betrifft Verbindungen der allgemeinen Formel I

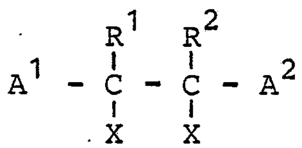

I,

in der

$A^2$ einen Rest $A^1$ oder einen Rest der Formel

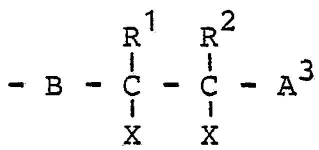

bedeutet, wobei

$A^1$ und $A^3$ unabhängig voneinander gegebenenfalls ein- oder mehrfach substituiertes Aryl oder Heteroaryl, Cyan, Carbonester, gegebenenfalls substituiertes Carbamoyl, Carboxyl, Alkanoyl oder Aroyl,

B eine direkte Bindung oder ein Brückenglied und

$R^1$ und $R^2$ unabhängig voneinander Wasserstoff, $C_1$- bis $C_4$-Alkyl, Aryl, Heteroaryl, Carboxyl, Cyan, Carbonester oder gegebenenfalls substituiertes Carbamoyl bedeuten, jeweils ein X Wasserstoff und das andere X ein Sulfonrest ist und wobei für $A^2$ gleich $A^1$ die beiden Reste $A^1$ gleich oder verschieden sein können.

Bg/P

Als Substituenten für die Arylreste $A^1$ und $A^3$ kommen z.B. Halogen, Alkyl, gegebenenfalls durch Hydroxy, Alkoxy oder Halogen substituiertes Alkyl, Alkoxy, Phenoxy, Carboxyl, Alkoxycarbonyl, gegebenenfalls substituiertes Carbamoyl, Cyan, gegebenenfalls substituiertes Amidino, Nitro, Amino, Acylamino, Triazinylamino, Hydroxysulfonyl, gegebenenfalls substituiertes Sulfonamid, Mercapto, gegebenenfalls substituiertes Alkyl-, Phenyl- oder Aralkylmercapto, gegebenenfalls substituiertes Alkyl-, Phenyl- oder Aralkylsulfoxid, gegebenenfalls substituiertes Alkyl-, Phenyl- oder Aralkylsulfon, phosphorhaltige Substituenten, Aryl, Alkanoyl, Aroyl, Azolyl oder Benzazolyl in Betracht.

Heteroarylreste $A^1$ und $A^3$ sind unabhängig voneinander ggf. substituiertes Benzimidazolyl, Benzoxazolyl, Benzthiazolyl, Thiazolyl, Oxazolyl, Oxdiazolyl, Isoxdiazolyl, Thiadiazolyl, Triazolyl, Benzofuranyl, Benzo[b]thiophenyl, Benzo-v-triazolyl, v-Triazolyl oder Pyridyl.

Einzelne Substituenten für die Arylreste sind neben den bereits genannten z.B. Fluor, Chlor, Brom, Methyl, Ethyl, Methoxy, Ethoxy, Methoxymethyl, Ethoxymethyl, Methoxyethyl, $-CO_2CH_3$, $-CO_2C_2H_5$, $-CO_2C_3H_7$, $-CO_2C_4H_9$, $-CO_2C_8H_{17}$,

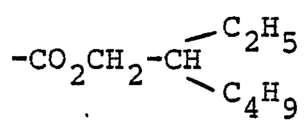, $-COOC_6H_5$, $-COOC_6H_4Cl$, $-COOC_6H_4OCH_3$,

$-CONH_2$, $-CONHCH_3$-, $-CONHC_2H_5$, $-CONHC_4H_9$,
$-CONHC_6H_5$, $-CON-(CH_3)_2$, $-CON(C_2H_5)_2$, $-CON(C_4H_9)_2$,
$-CONHC_2H_4N(CH_3)_2$, $-CONHC_3H_6N(CH_3)_2$, $-CONHC_3H_6N(C_2H_5)_2$,

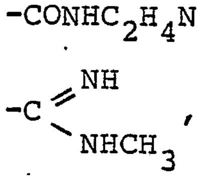, 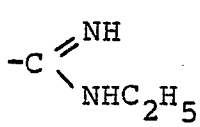, 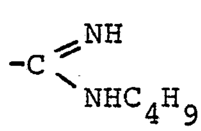, 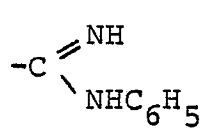, 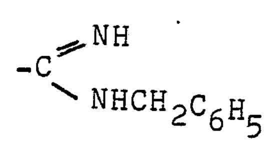,

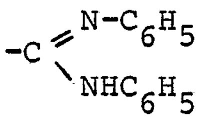, $-NHCOCH_3$, $-NHCOC_2H_5$, $-NHCOC_6H_5$, 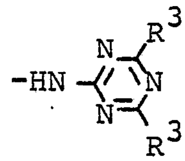,

$-SO_2NH_2$, $-SO_2NHCH_3$, $-SO_2NHC_2H_5$, $-SO_2NHC_3H_7$, $-SO_2NHC_4H_9$, $-SO_2NHC_6H_5$, $-SO_2NHCH_2C_6H_5$, $-SO_2N(CH_3)_2$, $-SO_2N(C_2H_5)_2$, $-SCH_3$, $-SC_2H_5$, $-SC_6H_5$, $-S-C_6H_4Cl$, $-SOCH_3$, $-SOC_2H_5$, $SOC_6H_5$, $-SOC_6H_4Cl$, $-SO_2CH_3$, $-SO_2C_2H_5$, $-SO_2C_6H_5$, $-SO_2CH_2C_6H_5$, $-SO_2C_6H_4Clp$, $-SO_2C_6H_4CH_3$, $-C_6H_5$ oder ein Rest der Formel

$-PO(OR)_2$, $-PO_3H$, $-P(O)-(C_6H_5)_2$, 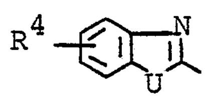, 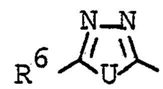,

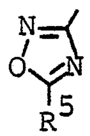, 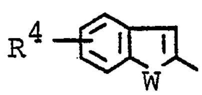, 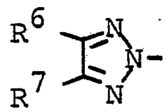, 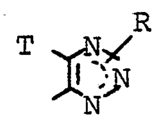, 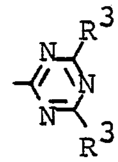, 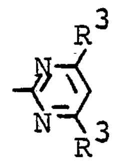,

wobei die Reste

$R^3$ unabhängig voneinander Fluor, Chlor, Brom, Hydroxy, Alkoxy, Aryloxy, Aralkoxy, Cycloalkoxy, Amino, Acylamino, Alkylamino, Dialkylamino, Alkyl- oder Arylmercapto, Alkyl oder Phenyl,

$R^4$ Wasserstoff, Halogen, $C_1$-$C_4$-Alkyl, Nitro, Hydroxy, Alkoxy, gegebenenfalls substituiertes Alkyl- oder Arylsulfon, Alkylmercapto, Arylmercapto oder Phenyl,

$R^5$ gegebenenfalls durch Halogen, Alkyl oder Nitro substituiertes Phenyl oder $C_1$- bis $C_8$-Alkyl,

U -O-, -S- oder -NR,

W O oder S,

T Cyan, gegebenenfalls substituiertes Carbamoyl, Carbalkoxy oder den Rest 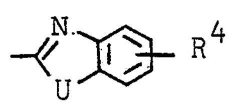,

$R^6$ und $R^7$ unabhängig voneinander Wasserstoff, Alkyl, Aryl oder Formyl,

$R^6$ und $R^7$ zusammen einen gegebenenfalls substituierten Benzring und

R Wasserstoff oder gegebenenfalls substituiertes Alkyl oder Aralkyl bedeuten.

Carbonesterreste $A^1$ und $A^2$ sind z. B.: $-CO_2CH_3$, $-CO_2C_2H_5$, $-CO_2C_3H_7$, $-CO_2C_4H_9$, $-CO_2C_5H_{11}$, $-CO_2C_6H_5$, $-CO_2C_2H_4OCH_3$, $-CO_2C_2H_4OC_2H_5$, $-CO_2C_2H_4OC_6H_5$, $-CO_2C_3H_7OCH_3$, $-CO_2C_3H_7OC_2H_5$, $-CO_2C_3H_7OC_6H_5$ oder $-CO_2$ 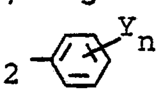, wobei

Y Halogen, $C_1$-$C_5$-Alkyl, Nitro, Amino, Acylamino, Di-$C_1$-$C_4$-Alkylamino, Hydroxy oder $C_1$-$C_4$-Alkoxy und

n 1, 2 oder 3 sind und bei mehreren Substituenten diese gleich oder verschieden sein können.

Einzelne Carbamoylreste $A^1$ und $A^2$ sind z.B.: $-CONH_2$, $-CONHCH_3$, $-CONHC_2H_5$, $-CONHC_4H_9$, $-CONHC_6H_6$, $-CONHC_6H_5$, $-CONH-CH_2-C_6H_5$, $-CON(CH_3)_2$, $-CON(C_2H_5)_2$, $-CON(C_4H_9)_2$, $-CONHC_2H_4N(CH_3)_2$, $-CONHC_3H_6N(CH_3)_2$, $-CONHC_3H_6N-(C_2H_5)_2$, $-\underset{CH_3}{\underset{|}{CON}}-C_6H_5$ oder $-CONH$ 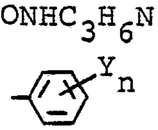 ,

wobei Y und n die bereits angegebene Bedeutung haben.

Einzelne Alkanoyl- und Aroylreste $A^1$ und $A^2$ sind z.B.: $-COCH_3$, $-COC_2H_5$, $-COC_3H_7$ oder $-CO$ 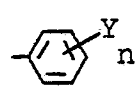, wobei Y und n die bereits angegebene Bedeutung haben.

Brückenglieder B sind z.B. gegebenenfalls substituiertes Phenylen, Biphenylen oder Polyphenylen sowie die Reste der Formeln

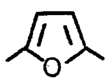 , 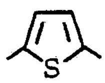 , 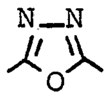 , 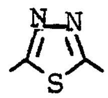 oder 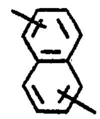 .

Einzelne Reste B sind neben den bereits genannten beispielsweise:

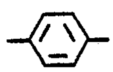 , 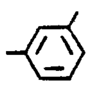 , 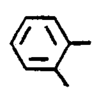 , 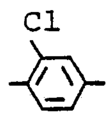 , 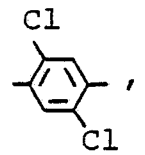 , 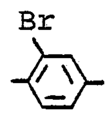 , 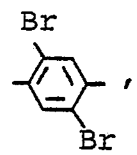 ,

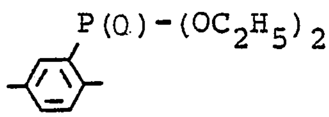 , 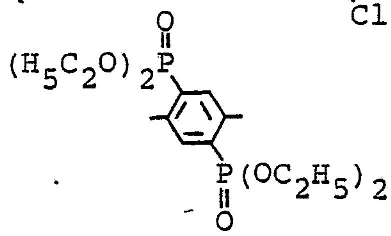 , 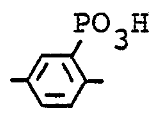 ,

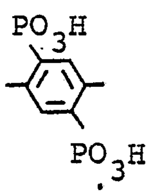 , 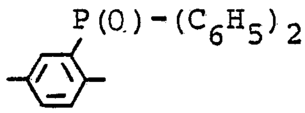 , 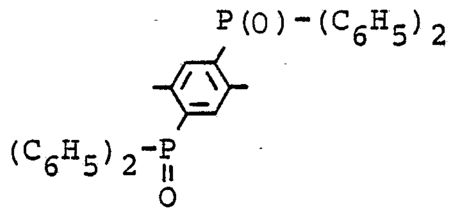 ,

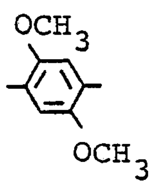 , 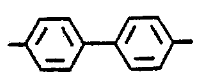 oder 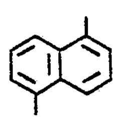 .

Einzelne Reste $R^1$ und $R^2$ sind außer den bereits erwähnten z.B.: Methyl, Ethyl, n-Propyl, n-Butyl, Phenyl, Benzoxazolyl, Benzimidazolyl oder Benzthiazolyl sowie die Reste der Formeln $-CO_2CH_3$, $-CO_2C_2H_5$, $-CO_2C_3H_7$, $-CONH_2$, $-CONHCH_3$, $-CONHC_2H_5$, $-CONHC_3H_7$, $-CONHC_4H_9$, $-CONHC_6H_5$,

$CONHCH_2C_6H_5$, $-CON(CH_3)_2$, $-CON(C_2H_5)_2$, $-CON(C_4H_9)_2$ oder $-CON(CH_2C_6H_5)CH_3$.

Sulfonreste X in der allgemeinen Formel I entsprechen der Formel $-SO_2Z$, wobei Z ein Alkyl, Aryl oder Aralkylrest ist.

Einzelne Reste Z sind z.B.: Methyl, Ethyl, Propyl, gegebenenfalls durch Chlor, Brom, Methyl, Ethyl, Nitro, Amino, Acylamino, Hydroxy, Methoxy oder Ethoxy substituiertes Phenyl oder Benzyl.

Von besonderer Bedeutung sind Verbindungen der Formel I, in der

$A^1$ und $A^3$ unabhängig voneinander gegebenenfalls ein- oder mehrfach durch Methyl, Ethyl, Methoxy, Ethoxy, Chlor, Brom, gegebenenfalls substituiertes Carbamoyl, Cyan, Carbalkoxy, Nitro, Amino, Acylamino, gegebenenfalls substituiertes Triazinylamino, Hydroxysulfonyl, gegebenenfalls substituiertes Sulfamoyl, gegebenenfalls substituiertes Alkyl-, Phenyl- oder Aralkylsulfon oder einen Rest

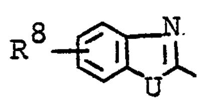 , 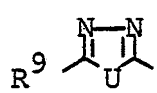 , 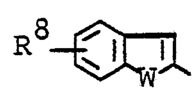 oder 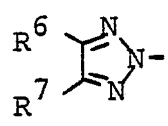

II III IV V

substituiertes Phenyl oder Reste der Formeln II bis V sind, wobei U, W, $R^6$ und $R^7$ die bereits angegebene Bedeutung haben und

$R^8$ Wasserstoff, Chlor, Brom, Methyl oder Methyl- oder Ethylsulfonyl,

$R^9$ Methyl, Ethyl oder gegebenenfalls durch Chlor, Brom, Methyl, Ethyl oder Nitro substituiertes Phenyl oder Pyridyl, ferner

$R^1$ und $R^2$ unabhängig voneinander Wasserstoff, $C_1$-$C_4$-Alkyl oder gegebenenfalls substituiertes Aryl, jeweils ein

X Wasserstoff und das andere $C_1$-$C_4$-Alkyl- oder Arylsulfon bedeuten und

B die angegebene Bedeutung hat.

Technisch besonders wertvoll sind Verbindungen der allgemeinen Formel I a

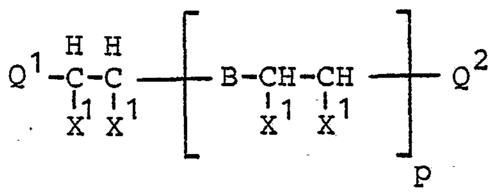

I a,

in der

$Q^1$ und $Q^2$ unabhängig voneinander gegebenenfalls durch Methyl, Ethyl, Methoxy, Ethoxy, Chlor, Brom, Hydroxymethyl, $C_1$- bis $C_4$-Alkoxymethyl, gegebenenfalls substituiertes Carbamoyl, Cyan, Carbalkoxy, Amino, Acylamino, gegebenenfalls substituiertes Triazinylamino, Hydroxysulfonyl oder einen der Reste der Formel II bis V substituiertes Phenyl oder ein Rest der Formeln II bis V, jeweils ein

$X^1$ Wasserstoff und das andere einen Rest der Formel

$$-SO_2Z^1$$ und

p 0 oder 1 sind, wobei

B die angegebene Bedeutung hat und

$Z^1$ gegebenenfalls ein- oder mehrfach durch Chlor, Brom, Methyl, Ethyl, Nitro, Amino, Acylamino, Hydroxy, Methoxy oder Ethoxy substituiertes Phenyl ist.

Zur Herstellung der Verbindungen der Formel I kann man Verbindungen der Formel VI

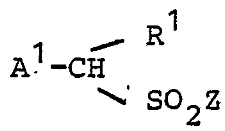 VI

mit Verbindungen der Formel VII

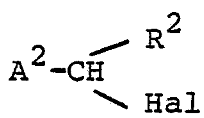 VII

umsetzen, wobei Hal Chlor oder Brom bedeutet.

Man kann auch Verbindungen der Formel VIII

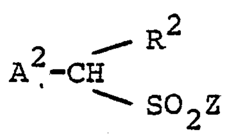 VIII

mit Verbindungen der Formel IX

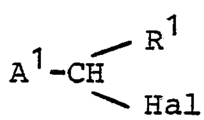 IX

umsetzen, wobei Hal Chlor oder Brom bedeutet.

Die Reaktion wird zweckmäßigerweise in indifferenten polaren aprotischen Lösungsmitteln unter Zuhilfenahme einer Base vorgenommen oder unter Phasentransferbedingungen unter Zuhilfenahme eines Phasentransferkatalysators.

Die Verbindungen der Formeln VI - IX sind teilweise bekannt oder sie können analog zu literaturbekannten Verbindungen hergestellt werden.

Einzelheiten der Darstellung der Verbindungen der Formel I können den Beispielen entnommen werden, in denen sich Angaben über Teile und Prozente, sofern nicht anders vermerkt, auf das Gewicht beziehen.

Die Verbindungen der Formel I sind wertvolle Zwischenprodukte, z.B. für die Farbstoff- und Pharmaindustrie, insbesondere zur Herstellung von fluoreszierenden Verbindungen.

Beispiel 1

13,8 Teile 4-Nitrobenzyl-phenyl-sulfon und 4,4 Teile p-Xylylendichlorid in 60 Volumenteilen Dimethylformamid werden innerhalb von 30 Minuten mit 9 Teilen einer 30 %igen Natriummethylat-Lösung in Methanol versetzt. Man rührt noch 30 Minuten bei Raumtemperatur und 60 Minuten bei 40 °C nach. Das Reaktionsgemisch wird dann auf Eiswasser gegossen, mit Eisessig auf pH 5 gestellt, der Feststoff abgesaugt und mit Wasser gewaschen. Man erhält 15,8 Teile der Verbindung

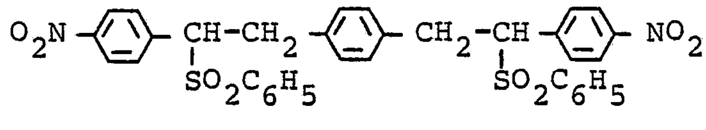

vom Schmelzpunkt 108 - 110 °C.

Beispiel 2

12,9 Teile 2-Cyanbenzyl-phenylsulfon in 50 Volumenteilen DMF werden mit 3 Volumenteilen 1,2-Dibromäthan versetzt. Man gibt 0,3 Teile Triäthylbenzylammoniumbromid und anschließend 10 Volumenteile Natriumhydroxid als 50 %ige Lösung in Wasser zu, wobei die Temperatur bis auf 45 °C ansteigt. Das Reaktionsgemisch wird dann 60 Minuten bei Raumtemperatur nachgerührt und anschließend auf Eiswasser gegossen. Man stellt mit Eisessig auf pH 5 ein und läßt über Nacht stehen. Das anfangs schmierig ausgefallene Reaktionsprodukt kristallisiert durch und wird abgesaugt. Man erhält 11,5 Teile der Verbindung der Formel

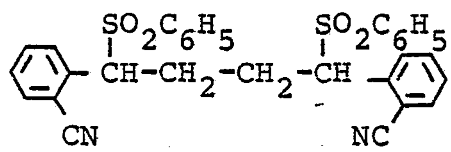

vom Schmelzpunkt 115 - 118 °C.

Beispiel 3

6,2 Teile der Verbindung der Formel

$$C_6H_5-SO_2-CH_2-CH_2-SO_2-C_6H_5$$

und 6,1 Teile o-Cyanbenzylchlorid in 30 Volumenteilen Dimethylsulfoxid werden mit 4,8 Volumenteilen einer 50 %igen NaOH-Lösung in Wasser versetzt. Man rührt 2 Stunden bei Raumtemperatur und 1 Stunde bei 50 °C nach und läßt dann über Nacht stehen. Das Reaktionsgemisch wird in Eiswasser

eingerührt, und das Reaktionsprodukt mit Methylenchlorid extrahiert. Die organische Phase wird über $Na_2SO_4$ getrocknet und eingeengt. Man erhält 14,7 Teile der Verbindung der Formel

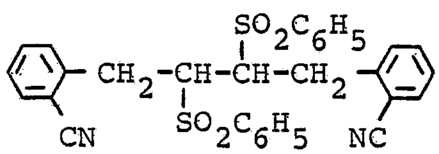

als flüssig-festen Rückstand, der mit Ethylether verrührt, einen Schmelzpunkt von 148 - 150 °C aufweist.

Beispiel 4

Zu 8,6 Teilen des Sulfons der Formel

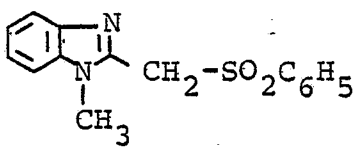

und 2,6 Teilen p-Xylylendichlorid in 60 Volumenteilen Dimethylformamid werden bei 20 - 25 °C 5,4 Teile Natriummethylat als 30 %ige Lösung in Methanol zugetropft. Man rührt noch 30 Minuten bei Raumtemperatur nach und gießt dann das Reaktionsgemisch auf Eiswasser. Der Niederschlag wird abgesaugt und mit Wasser gewaschen. Man erhält 9,5 Teile der Verbindung der Formel

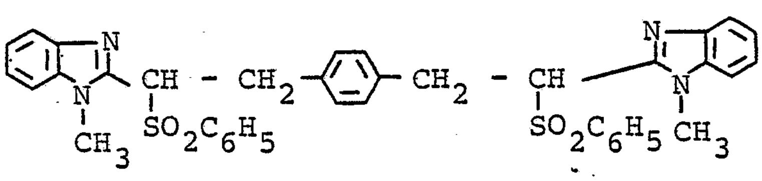

,

die einen Schmelzpunkt von über 300 °C besitzt.

Beispiel 5

Zu 8,6 Teilen der Verbindung der Formel

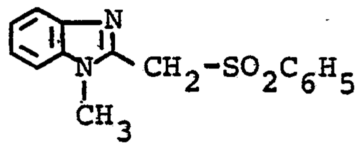

und 5,5 Teilen 2-Chloromethylbenzimidazol in 60 Volumenteilen Dimethylformamid werden bei 20 - 25 °C 5,4 Teile einer 30 %igen methanolischen Natriummethylat-Lösung innerhalb von 30 Minuten zugetropft. Man rührt noch 30 Minuten bei Raumtemperatur nach und gießt dann das Reaktionsgemisch auf Eiswasser. Der Niederschlag wird abgesaugt und mit Wasser gewaschen. Man erhält 10,2 Teile der Verbindung der Formel

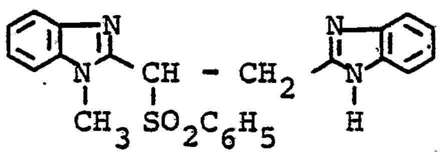 ,

vom Schmelzpunkt 162 - 165 °C.

Beispiel 6

Zu 10,3 Teilen 2-Cyanbenzyl-phenyl-sulfon und 7 Teilen p-Xylylendichlorid in 30 Volumenteilen Dimethylformamid werden innerhalb von 30 Minuten 7,2 Teile einer 30 %igen Natriummethylat-Lösung in Methanol bei 25 - 30 °C zugetropft. Man rührt noch 30 Minuten bei 30 - 40 °C nach und gießt das Reaktionsgemisch auf Eiswasser. Die ölige

Masse wird mit Methylenchlorid extrahiert, die organische Phase über $Na_2SO_4$ getrocknet und eingeengt. Man erhält 15,5 Teile der Verbindung der Formel

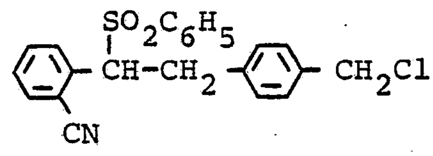

als öligen Rückstand, der ohne weitere Reinigung weiter umgesetzt werden kann.

Beispiel 7

7,9 Teile der Verbindung aus Beispiel 6 und 5,1 Teile 4-Cyanbenzyl-phenyl-sulfon werden in 30 Volumenteilen DMF gelöst. Bei 30 °C tropft man innerhalb von 30 Minuten 3,9 Teile einer 30 %igen Natriummethylat-Lösung in Methanol zu und läßt noch weitere 30 Minuten bei 30 °C und 30 Minuten bei 40 - 45 °C nachrühren. Der Fortlauf der Reaktion kann dünnschichtchromatographisch verfolgt werden. Nach Beendigung der Reaktion wird das Reaktionsgemisch in Eiswasser eingerührt, mit Essigsäure auf pH 6 gestellt und der Feststoff abgesaugt. Man erhält 12,8 Teile der Verbindung der Formel

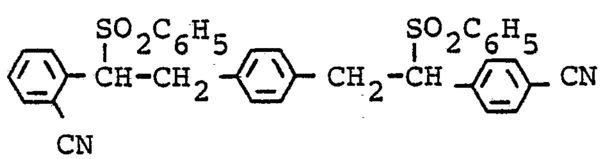
,

vom Schmelzpunkt 115 - 118 °C.

Beispiel 8

13,6 Teile der Verbindung

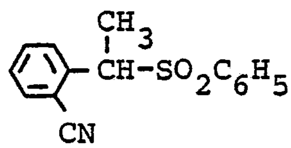

(hergestellt aus 2-Cyanbenzyl-phenyl-sulfon mit Methyljodid) und 4,4 Teile p-Xylylendichlorid werden in 50 Volumenteilen DMF bei 30 °C gelöst. Man tropft innerhalb von 30 Minuten 9 Teile Natriummethylat als 30 %ige methanolische Lösung zu und rührt 30 Minuten nach. Das Reaktionsgemisch wird dann in Eiswasser gegossen und 2 Stunden nachgerührt. Der ausgefallene Feststoff wird abgesaugt und mit Wasser gewaschen. Man erhält 13,9 Teile der Verbindung der Formel

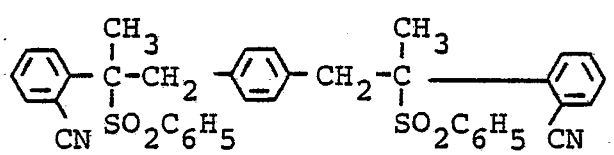 ,

vom Schmelzpunkt 119 - 122 °C.

Beispiel 9

12,3 Teile der Verbindung

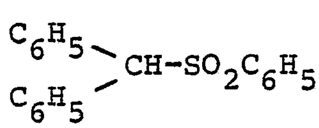

(hergestellt aus Diphenylchlormethan und Natriumbenzolsulfinat) und 3,5 Teile p-Xylylendichlorid in 40 Volumenteilen Dimethylformamid werden bei 0 - 10 °C mit 7,2 Teilen einer 30 %igen Natriummethylat-Lösung in Methanol innerhalb von 30 Minuten versetzt. Man rührt 2 Stunden bei 0 - 10 °C nach und läßt über Nacht stehen. Das Reaktionsgemisch wird dann in Eiswasser eingerührt, wobei das Reaktionsprodukt ausfällt. Man erhält 12,1 Teile der Verbindung der Formel

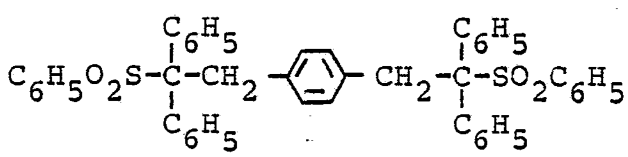 ,

vom Schmelzpunkt 170 - 172 °C.

Beispiel 10

8 Teile der Verbindung der Formel

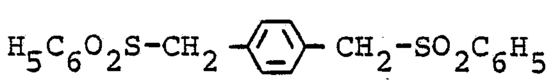

und 9,9 Teile o-Cyanbenzylchlorid werden in 30 Volumenteilen Dimethylformamid gelöst. Bei 30 °C tropft man 11,2 Teile einer 30 %igen Natriummethylat-Lösung in Methanol innerhalb von 30 Minuten zu und rührt anschließend noch 30 Minuten bei 30 °C und 60 Minuten bei 45 - 50 °C nach. Das Reaktionsgemisch wird dann auf Eiswasser gegossen und das Reaktionsprodukt mit Methylenchlorid extrahiert. Die organische Phase wird über $Na_2SO_4$

getrocknet und eingeengt. Man erhält 14 Teile der Verbindung der Formel

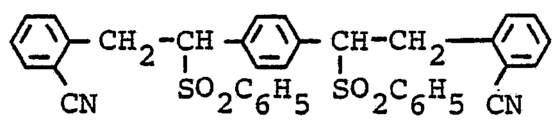

als öligen Rückstand, der nach einiger Zeit durchkristallisiert und einen Schmelzpunkt von 115 - 120 °C zeigt.

Beispiel 11

12,9 Teile o-Cyanbenzyl-phenyl-sulfon und 10 Teile ω-Bromacetophenon in 50 Volumenteilen Dimethylformamid werden bei 25 - 30 °C mit 9 Teilen einer 30 %igen Natriummethylat-Lösung in Methanol innerhalb von 30 Minuten versetzt. Man rührt anschließend 30 Minuten nach, gießt dann das Reaktionsgemisch auf Eiswasser und stellt mit Eisessig pH 5 ein. Nach 60 Minuten wird der Niederschlag abgesaugt und mit Wasser gewaschen. Man erhält 17,5 Teile der Verbindung der Formel

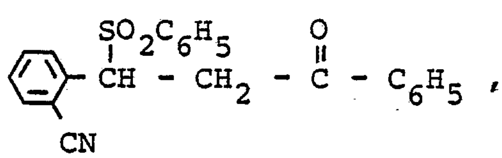

vom Schmelzpunkt 124 - 127 °C.

Analog lassen sich die in der Tabelle 1 aufgeführten Verbindungen herstellen.

Tabelle 1

$$Q^1 - \underset{\substack{|\\SO_2Z}}{CH} - CH_2 \left[ B - CH_2 - \underset{\substack{|\\SO_2Z}}{CH} \right]_p Q^2$$

| Beispiel Nr. | $Q^1$ | $Q^2$ | Z | p | B | Schmelzbereich °C |
|---|---|---|---|---|---|---|
| 12 | $C_6H_5$ | $C_6H_5$ | $C_6H_5$ | 1 | $C_6H_4$ | 180 - 186 |
| 13 | 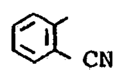 | wie $Q^1$ | " | " | " | 250 - 255 |
| 14 | 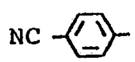 | wie $Q^1$ | " | " | " | 200 - 206 |
| 15 | 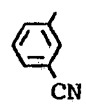 | wie $Q^1$ | " | " | " | 220 - 225 |
| 16 | 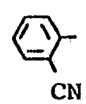 | wie $Q^1$ | 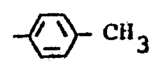 | " | " | 190 - 195 |
| 17 | 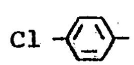 | wie $Q^1$ | $C_6H_5$ | " | " | 48 - 53 °C |

| Beispiel Nr. | $Q^1$ | $Q^2$ | Z | p | B | Schmelzbereich °C |
|---|---|---|---|---|---|---|
| 18 | 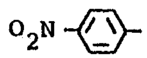 | wie $Q^1$ | $C_6H_5$ | 1 | $C_6H_4$ | 108 - 110 |
| 19 | 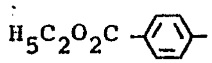 | wie $Q^1$ | " | " | " | 143 - 148 |
| 20 | 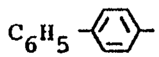 | wie $Q^1$ | " | " | " | 230 - 238 |
| 21 | 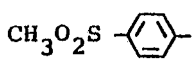 | wie $Q^1$ | " | " | 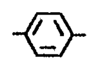 | 142 - 147 |
| 22 | 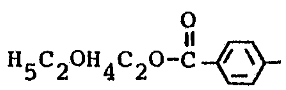 | wie $Q^1$ | 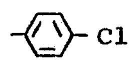 | " | " | 65 - 69 |
| 23 | 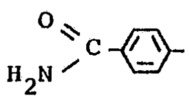 | wie $Q^1$ | 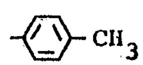 | " | " | 230 - 238 |
| 24 | 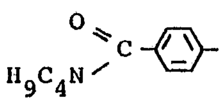 | wie $Q^1$ | $C_6H_5$ | " | " | 186 - 191 |
| 25 | 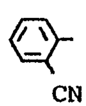 | wie $Q^1$ | 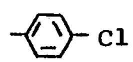 | " | " | 245 - 250 |

| Beispiel Nr. | $Q^1$ | $Q^2$ | Z | p | B | Schmelzbereich °C |
|---|---|---|---|---|---|---|
| 26 | 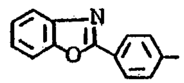 | wie $Q^1$ | $C_6H_5$ | 1 | 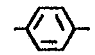 | 110 - 114 |
| 27 | 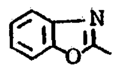 | wie $Q^1$ | " | " | " | 160 - 165 |
| 28 | 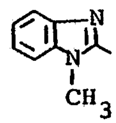 | wie $Q^1$ | " | " | " | über 300 |
| 29 | 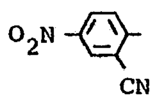 | wie $Q^1$ | " | " | " | 248 - 255 |
| 30 | 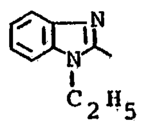 | wie $Q^1$ | 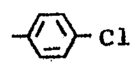 | " | " | 295 - 304 |
| 31 | 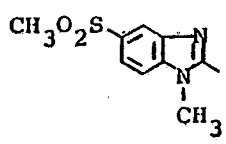 | wie $Q^1$ | $C_6H_5$ | " | " | über 300 |
| 32 | 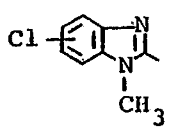 | wie $Q^1$ | " | " | " | über 300 |

| Beispiel Nr. | $Q^1$ | $Q^2$ | Z | p | B | Schmelzbereich °C |
|---|---|---|---|---|---|---|
| 33 | 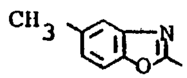 | wie $Q^1$ | 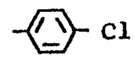 | 1 | 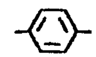 | 173 - 179 |
| 34 | 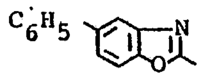 | wie $Q^1$ | $C_6H_5$ | " | " | 206 - 212 |
| 35 | 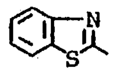 | wie $Q^1$ | " | " | " | 148 - 155 |
| 36 | 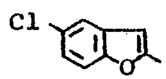 | wie $Q^1$ | " | " | " | 174 - 178 |
| 37 | 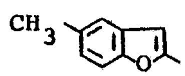 | wie $Q^1$ | " | " | " | 168 - 175 |
| 38 | 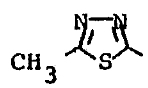 | wie $Q^1$ | " | " | " | $<35$ |
| 39 | 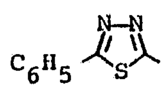 | wie $Q^1$ | " | " | " | 113 - 118 |
| 40 | 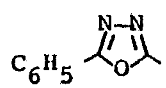 | wie $Q^1$ | " | " | " | 122 - 129 |

| Beispiel Nr. | $Q^1$ | $Q^2$ | Z | p | B | Schmelzbereich $^oC$ |
|---|---|---|---|---|---|---|
| 41 | 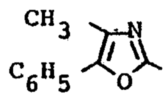 | wie $Q^1$ | $C_6H_5$ | 1 | 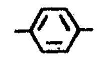 | 112 - 114 |
| 42 | 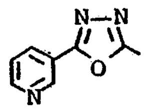 | wie $Q^1$ | " | " | " | 102 - 110 |
| 43 | 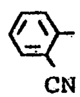 | 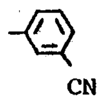 | " | " | " | 120 - 126 |
| 44 | 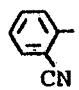 | 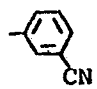 | 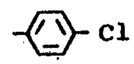 | " | " | 131 - 135 |
| 45 | " | 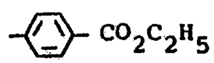 | " | " | " | 112 - 115 |
| 46 | 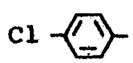 | 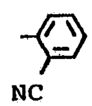 | $C_6H_5$ | " | " | 117 - 123 |
| 47 | 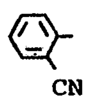 | " | " | " | 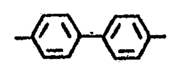 | 250 - 258 |
| 48 | 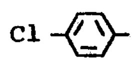 | " | " | " | " | 157 - 161 |

| Beispiel Nr. | $Q^1$ | $Q^2$ | Z | p | B | Schmelzbereich °C |
|---|---|---|---|---|---|---|
| 49 | 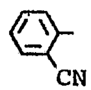 | 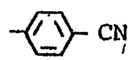 | $C_6H_5$ | 1 | 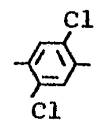 | 198 - 203 |
| 50 | 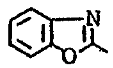 | " | " | " | " | 183 - 187 |
| 51 | 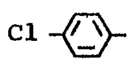 | 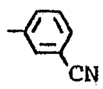 | " | " | " | 165 - 169 |
| 52 | " | " | " | " | 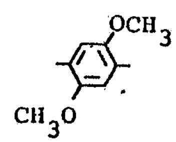 | 144 - 149 |
| 53 | 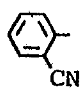 | 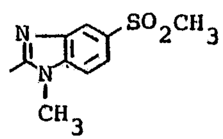 | " | " | 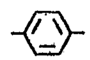 | 204 - 208 |
| 54 | " | 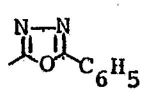 | " | " | " | 198 - 203 |
| 55 | 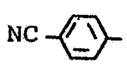 | 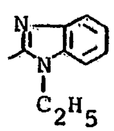 | " | " | " | 185 - 189 |

| Beispiel Nr. | $Q^1$ | $Q^2$ | Z | p | B | Schmelzbereich °C |
|---|---|---|---|---|---|---|
| 56 | 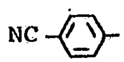 | 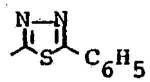 | $C_6H_5$ | 1 | 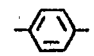 | 166 - 169 |
| 57 | " | 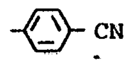 | " | " | 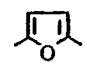 | 183 - 186 |
| 58 | 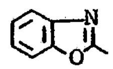 | wie $Q^1$ | " | " | " | 198 - 204 |
| 59 | 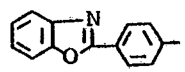 | 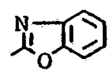 | " | " | " | 217 - 221 |
| 60 | 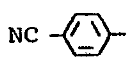 | wie $Q^1$ | " | " | 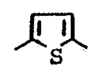 | 185 - 189 |
| 61 | $C_6H_5$ | wie $Q^1$ | " | " | - | 75 - 84 |
| 62 | 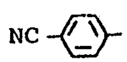 | wie $Q^1$ | " | " | - | 108 - 112 |
| 63 | " | wie $Q^1$ | 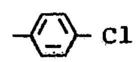 | " | - | 111 - 115 |
| 64 | 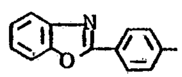 | wie $Q^1$ | $C_6H_5$ | " | - | 180 - 186 |

| Beispiel Nr. | $Q^1$ | $Q^2$ | Z | p | B | Schmelzbereich °C |
|---|---|---|---|---|---|---|
| 65 | 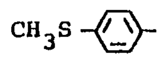 | wie $Q^1$ | $C_6H_5$ | 1 | - | 113 - 119 |
| 66 | 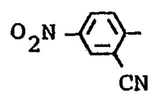 | wie $Q^1$ | " | 0 | - | 210 - 214 |
| 67 | 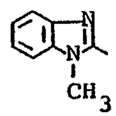 | wie $Q^1$ | " | 0 | - | 220 - 228 |
| 68 | 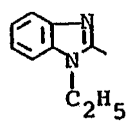 | 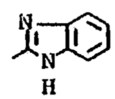 | " | 0 | - | 152 - 156 |
| 69 | 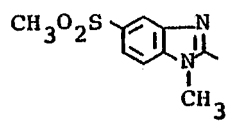 | wie $Q^1$ | " | 0 | - | 230 - 240 |
| 70 | " | 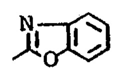 | " | 0 | - | 98 - 104 |
| 71 | 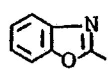 | 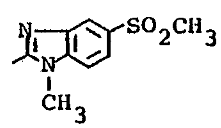 | " | 0 | - | 103 - 105 |

| Beispiel Nr. | $Q^1$ | $Q^2$ | Z | p | B | Schmelzbereich °C |
|---|---|---|---|---|---|---|
| 72 | 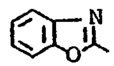 | 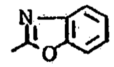 | $C_6H_5$ | 0 | - | 217 - 224 |
| 73 | 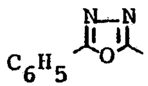 | 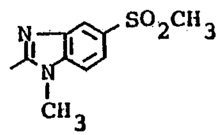 | " | 0 | - | 121 - 125 |
| 74 | 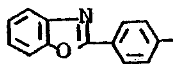 | 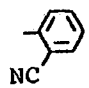 | " | 0 | - | 148 - 150 |
| 75 | " | 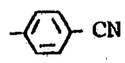 | " | 0 | - | 140 - 144 |
| 76 | 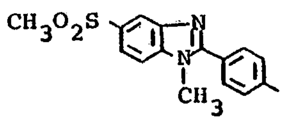 | 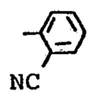 | " | 0 | - | 175 - 181 |
| 77 | 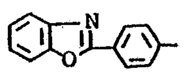 | 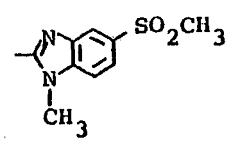 | " | 0 | - | 178 - 181 |
| 78 | " | 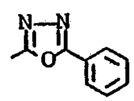 | " | 0 | - | 163 - 168 |
| 79 | 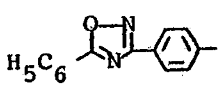 | 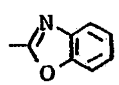 | " | 0 | - | 195 - 199 |

| Beispiel Nr. | $Q^1$ | $Q^2$ | Z | p | B | Schmelzbereich °C |
|---|---|---|---|---|---|---|
| 80 | 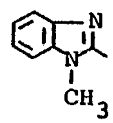 | 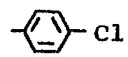 | $C_6H_5$ | O | - | 220 - 222 |
| 81 | " | 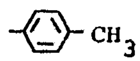 | " | " | - | 186 - 188 |
| 82 | " | $-CO_2CH_3$ | " | " | - | 165 - 169 |
| 83 | 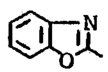 | 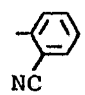 | " | " | - | 130 - 133 |

Patentansprüche

1. Sulfone der allgemeinen Formel

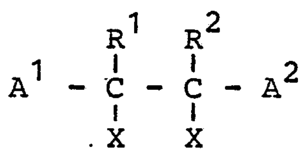 I,

in der

$A^2$ einen Rest $A^1$ oder einen Rest der Formel

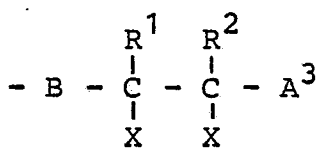

bedeutet, wobei

$A^1$ und $A^3$ unabhängig voneinander gegebenenfalls ein- oder mehrfach substituiertes Aryl oder Heteroaryl, Cyan, Carbonester, gegebenenfalls substituiertes Carbamoyl, Carboxyl, Alkanoyl oder Aroyl,

B eine direkte Bindung oder ein Brückenglied und

$R^1$ und $R^2$ unabhängig voneinander Wasserstoff, $C_1$- bis $C_4$-Alkyl, Aryl, Heteroaryl, Carboxyl, Cyan, Carbonester oder gegebenenfalls substituiertes Carbamoyl bedeuten, jeweils ein X Wasserstoff und das andere X ein Sulfonrest ist und wobei für $A^2$ gleich $A^1$ die beiden Reste $A^1$ gleich oder verschieden sein können.

2. Sulfone gemäß Anspruch 1, wobei in der Formel

$A^1$ und $A^3$ unabhängig voneinander gegebenenfalls ein- oder mehrfach durch Methyl, Ethyl, Methoxy, Ethoxy, Chlor, Brom, gegebenenfalls substituiertes Carbamoyl, Cyan, Carbalkoxy, Nitro, Amino, Acylamino, gegebenenfalls substituiertes Triazinylamino, Hydroxysulfonyl, gegebenenfalls substituiertes Sulfamoyl, gegebenenfalls substituiertes Alkyl-, Phenyl- oder Aralkylsulfon oder einen Rest

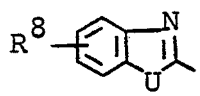 , 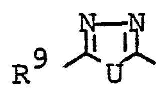 , 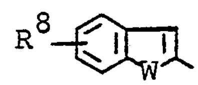 oder 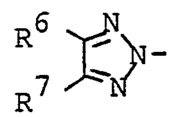

II III IV V

substituiertes Phenyl oder Reste der Formeln II bis V sind, wobei U, W, $R^6$ und $R^7$ die bereits angegebene Bedeutung haben und

$R^8$ Wasserstoff, Chlor, Brom, Methyl oder Methyl- oder Ethylsulfonyl,

$R^9$ Methyl, Ethyl oder gegebenenfalls durch Chlor, Brom, Methyl, Ethyl oder Nitro substituiertes Phenyl oder Pyridyl, ferner

$R^1$ und $R^2$ unabhängig voneinander Wasserstoff, $C_1$-$C_4$-Alkyl oder gegebenenfalls substituiertes Aryl, jeweils ein

X Wasserstoff und das andere $C_1$-$C_4$-Alkyl- oder Arylsulfon bedeuten und

B die angegebene Bedeutung hat.

3. Sulfone gemäß Anspruch 1 und 2 der allgemeinen Formel

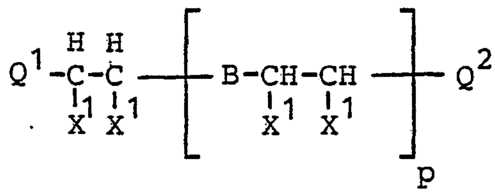

I a,

in der

$Q^1$ und $Q^2$ unabhängig voneinander gegebenenfalls durch Methyl, Ethyl, Methoxy, Ethoxy, Chlor, Brom, Hydroxymethyl, $C_1$- bis $C_4$-Alkoxymethyl, gegebenenfalls substituiertes Carbamoyl, Cyan, Carbalkoxy, Amino, Acylamino, gegebenenfalls substituiertes Triazinylamino, Hydroxysulfonyl oder einen der Reste der Formeln II bis V substituiertes Phenyl oder ein Rest der Formeln II bis V, jeweils ein

$X^1$ Wasserstoff und das andere einen Rest der Formel

$$-SO_2Z^1$$ und

p 0 oder 1 sind, wobei

B die angegebene Bedeutung hat und

$Z^1$ gegebenenfalls ein- oder mehrfach durch Chlor, Brom, Methyl, Ethyl, Nitro, Amino, Acylamino, Hydroxy, Methoxy oder Ethoxy substituiertes Phenyl ist.

4. Verwendung der Sulfone gemäß Anspruch 1 bis 3 als Zwischenprodukte zur Herstellung fluoreszierender Verbindungen.

Europäisches Patentamt

**EUROPÄISCHER RECHERCHENBERICHT**

0050225

Nummer der Anmeldung

EP 81 10 7483

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| X | CHEMICAL ABSTRACTS, Band 69, Nr. 19, 4. November 1968, Seiten 7170-7171, Nr. 76815q Columbus, Ohio, U.S.A. V. CALO et al.: "Addition of p-toluenesulfenyl chloride to diphenylacetylenes" & GAZZ. CHIM. ITAL. 1968, 98(5), 535-544 * Zusammenfassung * | 1-3 | C 07 C 147/06 147/107 149/30 C 07 D 521/00// (C 07 D 521/00 235/06 235/16 235/18 235/20 263/56 263/62 271/10 277/64 285/12 ./. 295/18 |
| X | CHEMICAL ABSTRACTS, Band 79, Nr. 13, 1. Oktober 1973, Seite 454, Nr. 78294z Columbus, Ohio, U.S.A. P. MESSINGER: "Sulfones as chemical transport forms of germicides. I. Addition of sulfinic acids to activated alkenes" & ARCH. PHARM. (WEINHEIM, GER.) 1973, 306(6), 458-462 * Zusammenfassung * | 1-3 | RECHERCHIERTE SACHGEBIETE (Int. Cl.³) C 07 C 147/107 147/103 147/06 C 07 D 263/56 277/64 307/79 285/12 271/10 263/32 235/20 235/06 307/38 333/18 413/06 413/04 |
| X | SYNTHESIS 1979, Seiten 461-463 Georg Thieme Verlag J. GOLINSKI et al.: "Reactions of organic anions; XCIV. Catalytic two-phase alkylation of benzyl sulfones and sulfonamides" * Seite 461, Verbindung 3d * | 1 | KATEGORIE DER GENANNTEN DOKUMENTE X: von besonderer Bedeutung allein betrachtet Y: von besonderer Bedeutung in Verbindung mit einer anderen Veröffentlichung derselben Kategorie A: technologischer Hintergrund O: nichtschriftliche Offenbarung P: Zwischenliteratur T: der Erfindung zugrunde liegende Theorien oder Grundsätze E: älteres Patentdokument, das jedoch erst am oder nach dem Anmeldedatum veröffentlicht worden ist D: in der Anmeldung angeführtes Dokument L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 20-01-1982 | GRAMAGLIA |

Europäisches Patentamt

**EUROPÄISCHER RECHERCHENBERICHT**

0050225

Nummer der Anmeldung

EP 81 10 7483

-2-

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der Maßgeblichen Teile | betrifft Anspruch | |
| | | | C 07 D 307/38<br>307/79<br>413/04<br>413/06<br>413/10) |
| | | | RECHERCHIERTE SACHGEBIETE (Int. Cl.3) |
| | | | |